# EUROPEAN PATENT SPECIFICATION

(11) **EP 3 524 280 B1**
(45) Date of publication and mention of the grant of the patent: **08.01.2020**
(21) Application number: 18156257.0
(22) Date of filing: 12.02.2018
(51) Int. Cl.: A61L 27/06, A61L 27/30, A61L 27/56, A61L 27/58

(54) **METHOD FOR PRODUCING A METALLIC IMPLANT**
VERFAHREN ZUR HERSTELLUNG EINES METALLISCHEN IMPLANTATS
PROCÉDÉ DE PRODUCTION D'UN IMPLANT MÉTALLIQUE

(43) Date of publication of application: 14.08.2019
(73) Proprietor: Helmholtz-Zentrum Geesthacht Zentrum für Material- und Küstenforschung GmbH, 21502 Geesthacht (DE); Element 22 GmbH, 24148 Kiel (DE)
(72) Inventor: Haramus, Vasyl, 21502 Geesthacht (DE); Ebel, Thomas, 21447 Handorf (DE); Ramakrishnegowda, Niranjan, 06108 Halle (DE); Bußacker, Sascha, 22453 Hamburg (DE); Schaper, Johannes Geronimo, 24852 Eggebek (DE)
(74) Representative: Uexküll & Stolberg

(56) References cited:
- WO-A2-2008/077263
- CN-A- 107 519 531
- DE-A1-102010 028 432

## Description

The present invention relates to a method for producing metallic implants which include both, biodegradable and biocompatible metals which are otherwise immiscible in proper connection, porosity and shape, as well as to medical devices produced therewith.

### BACKGROUND OF THE INVENTION

Metallic implants are widely used in orthopedic surgery and dental practice. Appropriate selection of the implant biomaterial is a key factor for long-term success of implants. The biologic environment does not accept completely any material. So to optimize biologic performance, implants should be selected to reduce the negative biologic response while maintaining adequate function. One of the key factors for maintaining adequate function is a fast and permanently tight connection between bone tissue and implant. Loosening of the implant is still often observed, and may make necessary additional surgical treatments combined with longtime recovery.

One of the causes for loosening of the implants may be a bacterial infection, which may occur even after the implant has been implemented in clean conditions. In order to avoid bacterial infection after the implant has been implemented, implants with a local drug treatment, which release the drug over a certain time period, have been proposed.

The coating of implants with ceramic hydroxylapatites (HA) or polymers has shown promising results for decreasing the risk of an infection. EP 2 259 805 B1 describes the method for gradient coating a Ti6Al4V substrate with HA or a polymer by applying a homogeneous sol-gel solution. The document also discloses addition of magnesium as stimulating agent to accelerate bone growth. However, the material tends to delaminate, and also drug loading is problematic.

Another cause for loosening of the implants may be poor adhesion between bone and implant. In order to strengthen the mechanical connection between bone and implants, to improve the adhesion there between, rough implant surfaces have been proposed. Furthermore, means which accelerate bone growth are desirable. Magnesium alloys which contain neodymium, yttrium, zirconium, and calcium disclosed in WO 2007/125532 A2 can be used for manufacturing implantable devices for medical treatment, such as orthopedic implants. The alloys have potential for the construction of monolithic, porous or multilayered structures which show good biocompatibility, acceptable mechanical properties and degradation rates. Due to the dominant fraction of magnesium in each of the layers, the magnesium alloys can be used only as temporary implants.

CN 107519531 A discloses a bone implant comprising a titanium alloy matrix, a porous metal powder coating and a bio-coating on the surface of the porous metal powder coating. The porous metal powder coating is obtained by spraying a mixture of titanium and magnesium powder on the surface of the titanium alloy substrate under argon gas protection.

It is therefore an object of the present invention to provide a flexible method for producing a metallic implant useful in orthopedic surgery and dental practice which has excellent mechanical properties, a structured surface and a porous, degradable layer on its surface. The porous, degradable layer shall be suitable for delivering drugs for a limited period of time, e.g. to avoid bacterial infection, and/or enhance bone growth and cell adhesion thereby promoting direct connection between bone and implant. It is also an object of the present invention to provide such metallic implant useful in orthopedic surgery and dental practice which has excellent mechanical properties, a structured surface and a porous, degradable layer on its surface.

### SUMMARY OF INVENTION

According to an embodiment the present invention relates to a method for producing shaped metal bodies useful as implant for orthopedic surgery or dental practice, comprising the steps of (a1)
providing a shaped metal body of titanium metal or a titanium alloy,
(b1) providing a mixture comprising magnesium powder or magnesium alloy powder and a polymeric binder,
(c1) preparing a shaped green part by positioning the shaped metal body into a mould and injecting the mixture comprising magnesium powder or magnesium alloy powder and a polymeric binder into the mould on top of the intermediate metal body (MIM) or by fused filament fabrication (FFF) of the second mixture onto the shaped intermediate metal body,
(d1) preparing a shaped brown part by subjecting the shaped green part to catalytic binder removal, solvent binder removal and/or thermal binder removal,
(e1) sintering the shaped brown part which has been subjected to catalytic, solvent and/or thermal binder removal to form a shaped metal body which includes a core of titanium metal or a titanium alloy, and which has a surface layer of a magnesium metal or of a magnesium alloy.

According to another embodiment the present invention relates to a method for producing shaped metal bodies useful as implant for orthopedic surgery or dental practice, comprising the steps of
(a2) providing a first mixture comprising titanium powder or titanium alloy powder and a polymeric binder,
(b2) preparing a first shaped green part by pressing the first mixture comprising titanium powder or titanium alloy powder and a polymeric binder into a first mould (MIM) or by fused filament fabrication of the first mixture (FFF),
(c2) preparing a first shaped brown part by subjecting the first shaped green part to catalytic binder removal, solvent binder removal and/or thermal binder removal,
(d2) preparing a shaped intermediate metal body by sintering the first shaped brown part which has been subjected to catalytic, solvent and/or thermal binder removal,
(b1) providing a second mixture comprising magnesium powder or magnesium alloy powder and a polymeric binder,
(c1) preparing a second shaped green part by positioning the shaped intermediate metal body into a second mould and injecting the second mixture comprising magnesium powder or magnesium alloy powder and a polymeric binder into the mould on top of the shaped intermediate metal body (MIM) or by fused filament fabrication (FFF) of the second mixture onto the shaped intermediate metal body,
(d1) preparing a second shaped brown part by subjecting the second shaped green part to catalytic binder removal, solvent binder removal and/or thermal binder removal,
(e1) sintering the second shaped brown part which has been subjected to catalytic, solvent and/or thermal binder removal to form a shaped metal body which includes a core of titanium metal or a titanium alloy, and which has a surface layer of a magnesium metal or of a magnesium alloy.

The method according to the present invention preferably makes use of metal injection moulding (MIM) which is a metalworking process by which finely-powdered metal is mixed with a measured amount of a binder material to comprise a "feedstock" capable of being handled by plastic processing equipment through a process known as injection moulding. The moulding process allows dilated (oversized due to the presence of binder) complex parts to be shaped in a single step. After moulding, the powder-binder mixture is subjected to steps that remove the binder (debinding), and sintering densifies the powders. Both, the core of the shaped metal bodies of titanium or a titanium alloy, and thereafter the magnesium or magnesium alloy coating are prepared by subsequent MIM steps. MIM is known and disclosed for example in US-Patent 4,197,118 and European Patent 2 292 806 B1.

Alternatively, the parts are shaped by fused filament fabrication (FFF), also referred to as fused filament fabrication (FFF), which is a method for creating three-dimensional objects as described in US patents 5,121,329; 5,340,433; 5,503,785; 5,866,058; 7,125,512; 7,255,821. The process includes producing three-dimensional objects by depositing repeated layers of solidifying material such as self-hardening waxes, thermoplastic resins, molten metals, two-part epoxies, foaming plastics, until the shape is formed, and as described e.g. in US-Patent 5,340, 433. The apparatus used for fused filament fabrication (FFF) typically comprises a moveable dispensing head having an elongated material passage therein connected to a dispensing outlet at one end thereof, and having an oppositely disposed material receiving end; a supply of material comprising a continuous flexible strand which solidifies at a predetermined temperature, and a material advance mechanism positioned to advance the strand into the elongated material passage on the head; a base member disposed in close, working proximity to said dispensing outlet of said dispensing head; mechanical means for moving said dispensing head and said base member relative to each other in multiple dimensions in a predetermined sequence and pattern; means for metering the discharge of said material in a fluid state from said dispensing outlet at a predetermined rate onto said base member to form a three-dimensional object as said dispensing head and base member are moved relative to each other; and heating means in heat exchange relation to said dispensing head for heating said strand material to a temperature above its solidification temperature in said material passage wherein said material is in a fluid state. In the present method, there is no need to melt the metal, as the metal particles are transported and adhered to the previous layer by action of the polymeric binder.

In the method according to the present invention, preparing of a first shaped green part may be accomplished either by MIM-technology or by FFF-technology, and preparing of the second shaped green part may also be accomplished either by MIM-technology or by FFF-technology, independently of whether for the preparation of the first shaped green part MIM-technology or FFF-technology was used.

The core of titanium metal or a titanium metal alloy can thus be produced using conventional metal injection moulding technology, as described for example in European Patent 2 292 806 B1, or fused filament fabrication, as described for example in US-Patent 5,340,433. The method according to the present invention is distinguished from the method described therein in that the surface of a magnesium metal or of a magnesium alloy is applied onto the core also making use of metal injection moulding or fused filament fabrication.

Preferably, the mixture comprising magnesium powder or magnesium alloy powder and a polymeric binder also comprises spaceholders such as an organic or an inorganic salt, more preferably an inorganic alkali metal or earth alkaline metal salt, such as NaCl, NaBr, KC1, KBr CaCl₂ and/or CaBr₂. Alternatively polymers such as PMMA may suitably be used as spaceholders. The spaceholder in the mixture occupies spaces during injection moulding or during fused filament fabrication, which will facilitate the provision of a defined porous structure of the magnesium layer. To achieve the porous structure of the magnesium layer, the spaceholder is removed from the green part, e.g. by dissolution, prior to debinding. The use of a spaceholder in the mixture makes it even possible to provide undercuts which will lead to a larger interface and improved mechanical connection between titanium part and magnesium part.

Also the mixture comprising titanium powder or titanium alloy powder and a polymeric binder may comprise the above spaceholders to roughen the surface of the first green parts to enhance adhesion of the magnesium metal or magnesium alloy. Preferably, however, a spaceholder such as an organic or an inorganic salt, more preferably an inorganic alkali metal or earth alkaline metal salt, such as NaCl, NaBr, KC1, KBr CaCl₂ and/or CaBr₂ is placed in the first mould prior to pressing the first mixture into a first mould (MIM), thereby providing pores and/or undercuts on the intermediate metal body.

### DETAILED DESCRIPTION OF THE INVENTION

With regard to the nature of the titanium alloy powder, particular preference is given to using titanium alloys which contain aluminium and/or vanadium as additional constituents. These additional alloy constituents such as aluminium and/or vanadium are in each case preferably present in an amount of from 2 to 10% by weight, based on the total weight of the alloy. A TiAl6V4 alloy containing about 6% by weight of aluminium, about 4% by weight of vanadium and titanium as balance is most preferred. Also preferred are β-titanium alloys containing e.g. up to 20% by weight niobium.

With regard to the nature of the magnesium alloy, particular preference is given to using magnesium alloys which contain calcium as additional constituent. The additional alloy constituents such as calcium are in each case preferably present in an amount of from 0.2 to 5% by weight, preferably 0.5 to 1.5% by weight, based on the total weight of the alloy. A Mg-0.9Ca alloy containing about 0.9% by weight of calcium and magnesium as balance is most preferred.

The particle size (maximum particle size, determined by sieving) of the titanium powder or titanium alloy powder, magnesium powder or magnesium alloy powder is preferably less than 50 µm, more preferably less than 45 µm, most preferably less than 25 µm.

The binder used for preparing the mixture comprising titanium powder or titanium alloy powder and a polymeric binder and for preparing the mixture comprising magnesium powder or magnesium alloy powder and a polymeric binder is preferably, independently selected from the group consisting of: polyamides, polyoxymethylene, polycarbonate, styrene-acrylonitrile copolymer, polyimide, natural waxes and oils, thermosets, cyanates, polypropylene, polyacetate, polyethylene, ethylene-vinyl acetate, polyvinyl alcohol, polyvinyl chloride, polystyrene, polymethyl methacrylate, anilines, mineral oils, water, agar, glycerol, polyvinylbutyryl, polybutyl methacrylate, cellulose, oleic acid, phthalates, paraffin waxes, carnauba wax, ammonium polyacrylate, digylceride stearate and oleate, glyceryl monostearate, isopropyl titanate, lithium stearate, monoglycerides, formaldehyde, octyl phosphate, olefin sulphonates, phosphate esters, stearic acid and mixtures and copolymers thereof, such as propylene-ethylene copolymers. Preference is given to using at least two binders, and the binder is most preferably composed of paraffin wax, polyethylene wax and stearic acid. The proportion by volume of the binder in the first and/or second mixture, respectively, is preferably less than 60%, more preferably from 20 to 50%. Most preferably, at least two binders are used for preparing the first mixture comprising titanium powder or titanium alloy powder and a polymeric binder, and most preferably a binder comprising propylene-ethylene copolymer is used for preparing the second mixture comprising magnesium powder or magnesium alloy powder and a polymeric binder.

As a further component in the first mixture comprising titanium powder or titanium alloy powder and a polymeric binder or in the second mixture comprising magnesium powder or magnesium alloy powder and a polymeric binder, preference is given to using an inorganic alkali metal or earth alkaline metal salt, such as NaCl, NaBr, KC1, KBr CaCl₂ and/or CaBr₂ or a polymer such as PMMA.

The mixing of titanium powder or titanium alloy powder and a polymeric binder and of magnesium powder or magnesium alloy powder and a polymeric binder is preferably, independently carried out in a kneader, extruder, rotation mixer or resonance mixer, and more preferably at a temperature of from 50 to 250°C, particularly preferably from 90 to 160°C.

The injection moulding, too, is preferably, independently carried out at a melt temperature of from 50 to 250°C, particularly preferably from 90 to 150°C, and preferably at a pressure of from 40 to 200 MPa.

Catalytic binder removal may be accomplished by chemical decomposition of the binder components using suitable chemicals, and then removing the decomposition products with a suitable solvent or thermally.

Solvent binder removal, sometimes referred-to as chemical binder removal (US 2008/0092383 A1, US 2014/0021660 A1, US 7,5581,498 B2), is accomplished by dissolution of the binder components in a suitable solvent. The solvent binder removal is preferably carried out in a hydrocarbon bath such as an aliphatic hydrocarbon bath, preferably in a pentane bath, a hexane bath or a heptane bath. The solvent binder removal is particularly preferably carried out in a hexane bath. The solvent binder removal is carried out at a temperature of preferably from 10 to 65°C, more preferably from 30 to 50°C.

The thermal binder removal is carried out at a temperature of less than 500°C, preferably from 200 to 450°C, and preferably under a reduced pressure of preferably from 2 to 50 mbar.

Sintering is preferably carried out at from 50 to 99% of the melting point of the metal or the metal alloy and more preferably in a protective gas or reducing atmosphere. More preferably thermal debinding and sintering is carried out successively using a temperature profile which comprises first heating the first or second shaped green part in a furnace to a debinding temperature, and, after debinding, further heating the so-obtained first or second shaped brown part, respectively, to a sintering temperature. The protective gas is preferably argon. Reducing atmosphere is preferably provided by hydrogen or a hydrogen-containing protective gas, such as an argon-hydrogen mixture. As an alternative, sintering can also be carried out under reduced pressure. In this case, the pressure is preferably from 10⁻¹ to 10⁻³ Pa (absolute). Thermal binder removal and sintering can advantageously take place in the same furnace. Suitable temperature programmes are preferably used for this purpose. In the thermal binder removal and/or in sintering, oxygen-binding material such as titanium powder or magnesium powder is preferably placed in the furnace to minimize the uptake of oxygen by the green parts.

The process of the invention is preferably carried out in such a way that the uptake of oxygen by the material to be sintered is less than 0.3% by weight. An oxygen content above about 0.3% by weight in the sintered shaped metal body may in some instances, e.g. when using Ti6Al4V, lead to embrittlement of the shaped metal body.

The invention is now described below in an exemplary manner, without restricting the general intent of the invention, based on exemplary embodiments with reference to the figures appended hereto, wherein:
**Fig. 1** depicts an exemplary temperature profile for the debinding step of a second shaped green part and subsequent sintering step of a second shaped brown part (temperature [degree centigrade, °C]; time [minutes, ']);
**Fig. 2** depicts a dog bone specimen prepared for investigation of the connection between Ti-part and Mg-0.9Ca-part depicted with an arrow;
**Figs. 3a** and 3**b** depict two dog bone specimen showing a Mg-Ti connection prepared according to an illustrative method similar to that defined in claim 1 of the present invention, wherein **Fig 3a** shows the connection made with a smooth interface, i.e. without the application of a salt in the Ti layer, whereas **Fig. 3b** shows the connection made with a rough interface, i.e. with the application of a salt in the Ti surface;
**Figs. 4a** and 4**b** depict enlarged photographs of the cross-sections of the connections between Ti and Mg-0.9Ca in the dog bone specimen of **Figs. 3a** (enlargement 200times) and **3b** (enlargement 100times), respectively;
**Fig. 5** depicts tensile tests carried out with two dog bone specimen made with a rough interface, where the lower graph depicts the tensile test carried out with combined Ti/Mg-0.9Ca sample while and the upper graph shows pure Mg-0.9Ca;

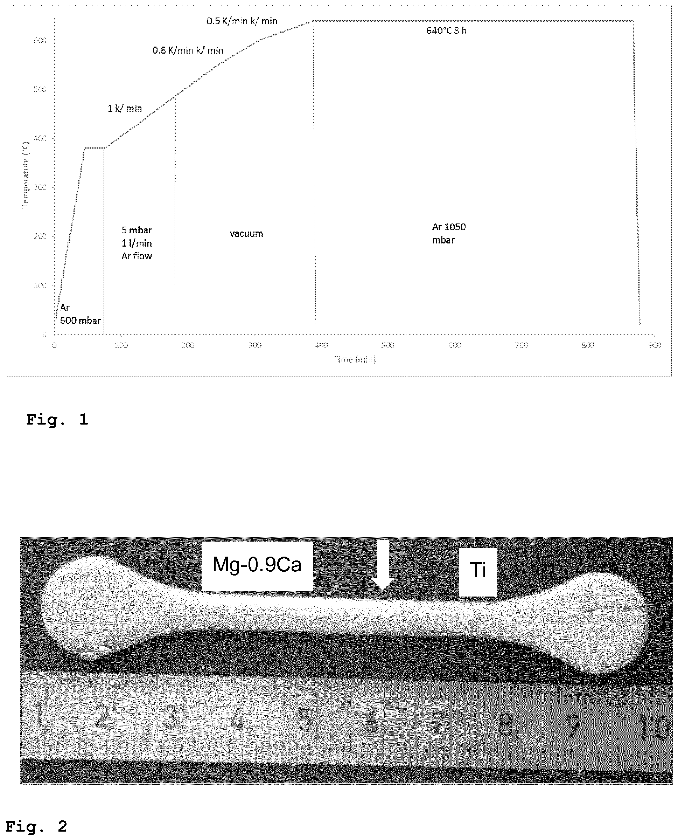

Referring to **Fig. 1****,** the second shaped green part contains a specimen comprising Mg-0.9Ca and a binder attached to a Ti surface. The second shaped green part was heated in a furnace from room temperature (20°C) to a debinding temperature of 380°C at a rate of 8 K/min. The pressure in the furnace was kept at 600 mbar while heating to the debinding temperature and debinding. The sample was kept approximately 35 minutes at the debinding temperature. Thereafter, the temperature was raised from 380°C to 480°C at a rate of 1 K/min while purging an argon gas flow at 1 L/min, thereby providing a second shaped brown part. The temperature was further raised to about 550°C at 1 K/min while now applying a vacuum, then to about 600°C at a rate of 0.8 K/min while still applying a vacuum, and finally to about 640°C at a rate of 0.5 K/min while still applying a vacuum. Thereafter the temperature was kept at 640°C for 8 h at a pressure of 105.0 kPa.

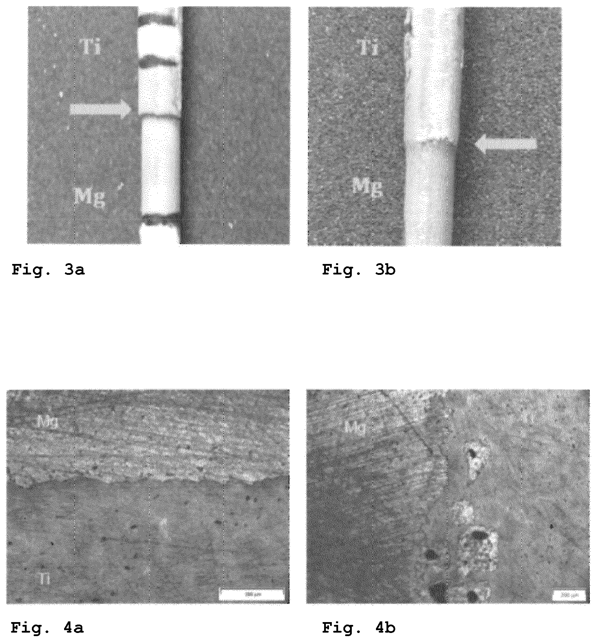

Referring now to **Fig. 3a****,** there is shown a dog bone specimen having a Mg-Ti connection prepared according to an illustrative method which is illustrative for that defined in claim 1 of the present invention, wherein the connection has been made with a smooth interface, i.e. without the application of a salt in the Ti layer. **Fig. 3b** shows a similar dog bone specimen having a Mg-Ti connection, wherein the connection has been made with a rough interface, i.e. with the application of a salt in the Ti layer. The difference between the method as claimed and the method for preparing the dog bone specimen is that instead of a coating, a first Ti half dog bone specimen has been prepared by using the MIM technology, and after placing the Ti half dog bone specimen into the dog bone mould, the other half part has been prepared using magnesium or magnesium alloy and a binder, and then following steps (g) and (h) according to claim 1. The specimens have been prepared to investigate the tightness of Ti-Mg connection when using MIM technology. Both samples show a tight connection between magnesium and titanium part, however, the transition between surfaces is optically smoother when a rough titanium interface has been applied.

This is further illustrated in **Figs. 4a** and **4b** which depict the Ti-Mg interface in enlargement.

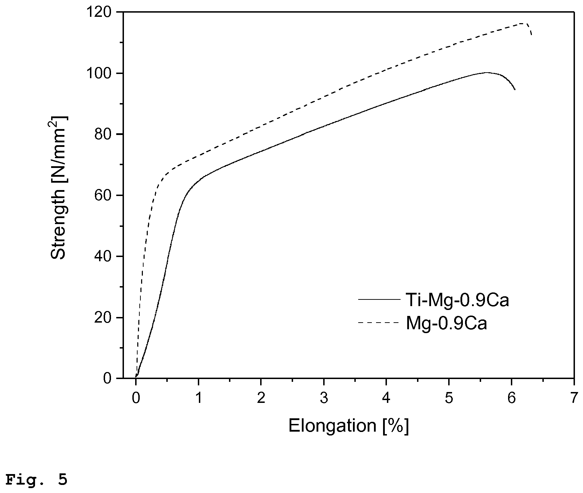

Referring now to **Fig. 5****,** there are shown tensile tests of Ti-Mg-0.9Ca dog bone specimen prepared using a rough interface of Ti and of dog bone specimen prepared only from Mg-0.9Ca at same conditions. It is shown that both specimen exhibit similar excellent tensile properties and can be elongated to about 6% without breaking. The strength of the connection between Ti and Mg-0.9Ca is enough to keep tensile properties of specimen as whole.

The present invention thus provides a novel method for producing shaped metal bodies useful as implant for orthopedic surgery or dental practice. The method yields shaped metal bodies having a titanium or titanium alloy core thoroughly or partially coated with magnesium or a magnesium alloy.

## Claims

1. A method for producing shaped metal bodies useful as implant for orthopedic surgery or dental practice, comprising the steps of
(a2) providing a shaped metal body of titanium metal or a titanium alloy,
(b2) providing a mixture comprising magnesium powder or magnesium alloy powder and a polymeric binder,
(c2) preparing a shaped green part by positioning the shaped metal body of titanium metal or a titanium alloy into a mould and injecting the mixture comprising magnesium powder or magnesium alloy powder and a polymeric binder into the mould on top of the intermediate metal body (MIM) or by fused filament fabrication (FFF) of the second mixture onto the shaped intermediate metal body,
(d2) preparing a shaped brown part by subjecting the shaped green part to catalytic binder removal, solvent binder removal and/or thermal binder removal,
(e2) sintering the shaped brown part which has been subjected to catalytic, solvent and/or thermal binder removal to form a shaped metal body which includes a core of titanium metal or a titanium alloy, and which has a surface layer of a magnesium metal or of a magnesium alloy.

2. The method of claim 1, wherein the mixture comprising magnesium powder or magnesium alloy powder and a polymeric binder, further comprises an inorganic alkali metal or earth alkaline metal salt, and the inorganic salt is removed from the first or second green part, respectively, prior to debinding.

3. A method for producing shaped metal bodies useful as implant for orthopedic surgery or dental practice, comprising the steps of
(a1) providing a first mixture comprising titanium powder or titanium alloy powder and a polymeric binder,
(b1) preparing a first shaped green part by pressing the first mixture comprising titanium powder or titanium alloy powder and a polymeric binder into a first mould (MIM) or by fused filament fabrication of the first mixture (FFF),
(c1) preparing a first shaped brown part by subjecting the first shaped green part to catalytic binder removal, solvent binder removal and/or thermal binder removal,
(d1) preparing a shaped intermediate metal body by sintering the first shaped brown part which has been subjected to catalytic, solvent and/or thermal binder removal,
(b2) providing a second mixture comprising magnesium powder or magnesium alloy powder and a polymeric binder,
(c2) preparing a second shaped green part by positioning the shaped intermediate metal body into a second mould and injecting the second mixture comprising magnesium powder or magnesium alloy powder and a polymeric binder into the mould on top of the shaped intermediate metal body (MIM) or by fused filament fabrication (FFF) of the second mixture onto the shaped intermediate metal body,
(d2) preparing a second shaped brown part by subjecting the second shaped green part to catalytic binder removal, solvent binder removal and/or thermal binder removal,
(e2) sintering the second shaped brown part which has been subjected to catalytic, solvent and/or thermal binder removal to form a shaped metal body which includes a core of titanium metal or a titanium alloy, and which has a surface layer of a magnesium metal or of a magnesium alloy.

4. The method of claim 3, wherein the first mixture comprising titanium powder or titanium alloy powder and a polymeric binder, and/or the second mixture comprising magnesium powder or magnesium alloy powder and a polymeric binder, further comprises an inorganic alkali metal or earth alkaline metal salt, and the inorganic salt is removed from the first or second green part, respectively, prior to debinding.

5. The method of any one of the previous claims, wherein the titanium alloy contains aluminium, vanadium and/or niobium as additional constituents.

6. The method of any one of the previous claims, wherein the magnesium alloy contains calcium as an additional constituent.

7. The method of any one of the previous claims, wherein the polymeric binder is independently selected from the group consisting of: polyamides, polyoxymethylene, polycarbonate, styrene-acrylonitrile copolymer, polyimide, natural waxes and oils, thermosets, cyanates, polypropylene, polyacetate, polyethylene, ethylene-vinyl acetate, polyvinyl alcohol, polyvinyl chloride, polystyrene, polymethyl methacrylate, anilines, mineral oils, water, agar, glycerol, polyvinylbutyryl, polybutyl methacrylate, cellulose, oleic acid, phthalates, paraffin waxes, carnauba wax, ammonium polyacrylate, digylceride stearate and oleate, glyceryl monostearate, isopropyl titanate, lithium stearate, monoglycerides, formaldehyde, octyl phosphate, olefin sulphonates, phosphate esters, stearic acid and mixtures and copolymers thereof. Preference is given to using at least two binders, and the binder is most preferably composed of paraffin wax, polyethylene wax and stearic acid.

8. The method of any one of the previous claims, wherein thermal binder removal is carried out at a temperature of less than 500°C.

9. The method of any one of the previous claims, wherein sintering is carried out at from 50 to 99% of the melting point of the metal or the metal alloy.

10. The method of any one of claims 3 to 9, wherein mixing of titanium powder or titanium alloy powder and a polymeric binder; and/or of magnesium powder or magnesium alloy powder and a polymeric binder is preferably, independently carried out in a kneader, extruder, rotation mixer or resonance mixer.

11. The method of claim 10, wherein the mixing is carried out at a temperature of from 50 to 250°C.

12. The method of any one of the previous claims, wherein injection moulding is, independently, carried out at a melt temperature of from 50 to 250°C.

13. The method of any one of the previous claims, wherein injection moulding is, independently, carried out at a pressure of from 40 to 200 MPa.

## Patentansprüche

1. Verfahren zur Herstellung von Metallformkörpern, die als Implantate für die orthopädische Chirurgie oder Zahnmedizinischen Praxis verwendbar sind, umfassend die Schritte:
(a2) Bereitstellen eines Metallformkörpers aus Titanmetall oder einer Titanlegierung,
(b2) Bereitstellen einer Mischung umfassend Magnesiumpulver oder Magnesiumlegierungspulver und ein polymeres Bindemittel,
(c2) Herstellen eines geformten Grünteils durch Einbringen des Metallformkörpers aus Titanmetall oder einer Titanlegierung in eine Form und Spritzgießen der Mischung umfassend Magnesiumpulver oder Magnesiumlegierungspulver und ein polymeres Bindemittel in die Form auf den Metallzwischenformkörper (MIM) oder mittels Schmelzschichtverfahren (Fused Filament Fabrication, FFF) der zweiten Mischung auf den Metallzwischenformkörper,
(d2) Herstellen eines geformten Braunteils, indem das Grünteil einer katalytischen Entbinderung und/oder einer Lösungsmittel-Entbinderung und/oder einer thermischen Entbinderung unterworfen wird,
(e2) Sintern des geformten Braunteils, das einer katalytischen Entbinderung und/oder einer Lösungsmittel-Entbinderung und/oder einer thermischen Entbinderung unterworfen wurde, um einen Metallformkörper zu bilden, der einen Kern aus Titanmetall oder einer Titanlegierung enthält und der eine Oberflächenschicht aus einem Magnesiummetall oder Magnesiumlegierung aufweist.

2. Verfahren nach Anspruch 1, wobei die Mischung, die Magnesiumpulver oder Magnesiumlegierungspulver und ein polymeres Bindemittel umfasst, weiterhin ein anorganisches Alkalimetall- oder Erdalkalimetallsalz umfasst und das anorganische Salz aus dem ersten bzw. zweiten Grünteil vor der Entbinderung entfernt wird.

3. Verfahren zur Herstellung von Metallformkörpern, die als Implantate für die orthopädische Chirurgie oder Zahnmedizinischen Praxis verwendbar sind, umfassend die Schritte:
(a1) Bereitstellen einer ersten Mischung umfassend Titanpulver oder Titanlegierungspulver und ein polymeres Bindemittel,
(b1) Herstellen eines ersten geformten Grünteils durch Pressen der ersten Mischung umfassend Titanpulver oder Titanlegierungspulver und ein polymeres Bindemittel in eine erste Form (MIM) oder mittels Schmelzschichtverfahren (Fused Filament Fabrication, FFF),
(c1) Herstellen eines ersten geformten Braunteils, indem das Grünteil einer katalytischen Entbinderung und/oder einer Lösungsmittel-Entbinderung und/oder einer thermischen Entbinderung unterworfen wird,
(d1) Herstellen eines Metallzwischenformkörpers durch Sintern des ersten geformten Braunteils, das einer katalytischen Entbinderung und/oder einer Lösungsmittel-Entbinderung und/oder einer thermischen Entbinderung unterworfen wurde,
(b2) Bereitstellen einer zweiten Mischung umfassend Magnesiumpulver oder Magnesiumlegierungspulver und ein polymeres Bindemittel,
(c2) Herstellen eines zweiten geformten Grünteils durch Einbringen des Metallzwischenformkörpers in eine zweite Form und Spritzgießen der zweiten Mischung umfassend Magnesiumpulver oder Magnesiumlegierungspulver und ein polymeres Bindemittel in die Form auf den Metallzwischenformkörper (MIM) oder mittels Schmelzschichtverfahren (Fused Filament Fabrication, FFF) der zweiten Mischung auf den Metallzwischenformkörper,
(d2) Herstellen eines zweiten geformten Braunteils, indem das zweite Grünteil einer katalytischen Entbinderung und/oder einer Lösungsmittel-Entbinderung und/oder einer thermischen Entbinderung unterworfen wird,
(e2) Sintern des zweiten geformten Braunteils, das einer katalytischen Entbinderung und/oder einer Lösungsmittel-Entbinderung und/oder einer thermischen Entbinderung unterworfen wurde, um einen Metallformkörper zu bilden, der einen Kern aus Titanmetall oder einer Titanlegierung enthält und der eine Oberflächenschicht aus einem Magnesiummetall oder Magnesiumlegierung aufweist.

4. Verfahren nach Anspruch 3, wobei die erste Mischung, die Titanpulver oder Titanlegierungspulver und ein polymeres Bindemittel umfasst, und/oder die zweite Mischung, die Magnesiumpulver oder Magnesiumlegierungspulver und ein polymeres Bindemittel umfasst, weiterhin ein anorganisches Alkalimetall- oder Erdalkalimetallsalz umfasst und das anorganische Salz aus dem ersten bzw. zweiten Grünteil vor der Entbinderung entfernt wird.

5. Verfahren nach einem der vorhergehenden Ansprüche, wobei die Titanlegierung Aluminium, Vanadium und/oder Niob als zusätzliche Bestandteile enthält.

6. Verfahren nach einem der vorhergehenden Ansprüche, wobei die Magnesiumlegierung Calcium als einen zusätzlichen Bestandteil enthält.

7. Verfahren nach einem der vorhergehenden Ansprüche, wobei das polymere Bindemittel unabhängig ausgewählt ist aus der Gruppe bestehend aus: Polyamiden, Polyoxymethylen, Polycarbonat, Styrol-Acrylnitril-Copolymer, Polyimid, natürlichen Wachsen und Ölen, Duroplasten, Cyanaten, Polypropylen, Polyacetat, Polyethylen, Ethylenvinylacetat, Polyvinylalkohol, Polyvinylchlorid, Polystyrol, Polymethylmethacrylat, Aniline, Mineralöle, Wasser, Agar, Glycerin, Polyvinylbutyryl, Polybutylmethacrylat, Cellulose, Ölsäure, Phthalate, Paraffinwachse, Carnaubawachs, Ammoniumpolyacrylat, Digylcerid Stearat und Oleat, Glycerylmonostearat, Isopropyltitanat, Lithiumstearat, Monoglyceride, Formaldehyd, Octylphosphat, Olefinsulfonate, Phosphatester, Stearinsäure und Gemischen und Copolymeren davon. Bevorzugt werden mindestens zwei Bindemittel eingesetzt, wobei das Bindemittel besonders bevorzugt aus Paraffinwachs, Polyethylenwachs und Stearinsäure besteht.

8. Verfahren nach einem der vorhergehenden Ansprüche, wobei die thermische Entbinderung bei einer Temperatur von weniger als 500°C durchgeführt wird

9. Verfahren nach einem der vorhergehenden Ansprüche, wobei das Sintern bei 50 bis 99% des Schmelzpunktes des Metalls oder der Metalllegierung durchgeführt wird.

10. Verfahren nach einem der Ansprüche 3 bis 9, wobei das Mischen von Titanpulver oder Titanlegierungspulver und einem polymeren Bindemittel; und/oder aus Magnesiumpulver oder Magnesiumlegierungspulver und einem polymeren Bindemittel vorzugsweise unabhängig in einem Kneter, Extruder, Rotationsmischer oder Resonanzmischer durchgeführt wird.

11. Verfahren nach Anspruch 10, wobei das Mischen bei einer Temperatur von 50 bis 250°C durchgeführt wird.

12. Verfahren nach einem der vorhergehenden Ansprüche, wobei das Spritzgießen unabhängig bei einer Schmelztemperatur von 50 bis 250°C durchgeführt wird.

13. Verfahren nach einem der vorhergehenden Ansprüche, wobei das Spritzgießen unabhängig bei einem Druck von 40 bis 200 MPa durchgeführt wird.

## Revendications

1. Procédé de production de corps métalliques façonnés utilisés comme implant pour la chirurgie orthopédique ou la pratique dentaire, comprenant les étapes consistant à :
(a2) prendre un corps métallique façonné fait de métal de titane ou d'un alliage de titane,
(b2) prévoir un mélange comprenant une poudre de magnésium ou poudre d'alliage de magnésium et un liant polymère,
(c2) préparer une pièce verte façonnée en positionnant le corps métallique façonné fait de métal de titane ou d'un alliage de titane dans un moule et injecter le mélange comprenant la poudre de magnésium ou la poudre d'alliage de magnésium et un liant polymère dans le moule sur le dessus du corps métallique intermédiaire (MIM) ou par fabrication par dépôt de filament fondu (FFF) du second mélange sur le corps métallique façonné intermédiaire,
(d2) préparer une pièce brune façonnée en soumettant la pièce verte façonnée à une élimination du liant par voie catalytique, élimination du liant au solvant et/ou élimination du liant par voie thermique,
(e2) fritter la pièce brune façonnée qui a été soumise à une élimination du liant par voie catalytique, élimination du liant au solvant et/ou élimination du liant par voie thermique, pour former un corps métallique façonné qui comprend un noyau en métal de titane ou alliage de titane, et qui a une couche de surface faite d'un métal de magnésium ou d'un alliage de magnésium.

2. Procédé selon la revendication 1, dans lequel le mélange comprenant une poudre de magnésium ou une poudre d'alliage de magnésium et un liant polymère, comprend en outre un sel inorganique de métal alcalin ou de métal alcalino-terreux et le sel inorganique est retiré de la première ou de la seconde pièce verte, respectivement, avant le déliantage.

3. Procédé de production de corps métalliques façonnés utilisés comme implant pour la chirurgie orthopédique ou la pratique dentaire, comprenant les étapes consistant à :
(a1) prévoir un premier mélange comprenant une poudre de titane ou une poudre d'alliage de titane et un liant polymère,
(b1) préparer une première pièce verte façonnée en comprimant le premier mélange comprenant une poudre de titane ou une poudre d'alliage de titane et un liant polymère dans un premier moule (MIM) ou par fabrication par dépôt de filament fondu du premier mélange (FFF),
(c1) préparer une première pièce brune façonnée en soumettant la première pièce verte façonnée à une élimination du liant par voie catalytique, élimination du liant au solvant et/ou élimination du liant par voie thermique,
(d1) préparer un corps métallique intermédiaire façonné en frittant la première pièce brune façonnée qui a été soumise à une élimination du liant par voie catalytique, élimination du liant au solvant et/ou élimination du liant par voie thermique
(b2) prévoir un second mélange comprenant une poudre de magnésium ou une poudre d'alliage de magnésium et un liant polymère,
(c2) préparer une seconde pièce verte façonnée en positionnant le corps métallique intermédiaire façonné dans un second moule et injecter le second mélange comprenant une poudre de magnésium ou une poudre d'alliage de magnésium et un liant polymère dans le moule sur le dessus du corps métallique intermédiaire façonné (MIM) ou par fabrication par de dépôt de filament fondu (FFF) du second mélange sur le corps métallique intermédiaire façonné,
(d2) préparer une seconde pièce brune façonnée en soumettant la seconde pièce verte façonnée à une élimination du liant par voie catalytique, élimination du liant par solvant et/ou élimination du liant par voie thermique,
(e2) fritter la seconde pièce brune façonnée qui a été soumise à une élimination du liant par voie catalytique, par solvant et/ou par voie thermique, pour former un corps métallique façonné qui comprend un noyau en métal de titane ou alliage de titane, et qui a une couche de surface faite d'un métal de magnésium ou d'un alliage de magnésium.

4. Procédé selon la revendication 3, dans lequel le premier mélange comprenant une poudre de titane ou une poudre d'alliage de titane et un liant polymère, et/ou le second mélange comprenant une poudre de magnésium ou une poudre d'alliage de magnésium et un liant polymère, comprend en outre un sel inorganique de métal alcalin ou de métal alcalino-terreux, et le sel inorganique est retiré de la première ou de seconde pièce verte, respectivement, avant le déliantage.

5. Procédé selon l'une quelconque des précédentes revendications, dans lequel l'alliage de titane contient de l'aluminium, du vanadium et/ou du niobium comme constituants additionnels.

6. Procédé selon l'une quelconque des précédentes revendications, dans lequel l'alliage de magnésium contient du calcium comme constituant additionnel.

7. Procédé selon l'une quelconque des précédentes revendications, dans lequel le liant polymère est sélectionné indépendamment parmi le groupe comprenant : les polyamides, le polyoxyméthylène, le polycarbonate, un copolymère de styrène-acrylonitrile, le polyimide, les cires et huiles naturelles, les thermodurcissants, les cyanates, le polypropylène, le polyacétate, le polyéthylène, l'éthylène-acétate de vinyle, l'alcool polyvinylique, le polychlorure de vinyle, le polystyrène, le polyméthacrylate de méthyle, les anilines, les huiles minérales, l'eau, l'agar, le glycérol, le poly(butyryl vinylique), le méthacrylate de polybutyle, la cellulose, l'acide oléique, les phtalates, les cires de paraffine, la cire de carnauba, le polyacrylate d'ammonium, le stéarate et oléate de diglycéride, le monostéarate de glycéryle, le titanate d'isopropyle, le stéarate de lithium, les monoglycérides, le formaldéhyde, le phosphate d'octyle, les sulfonates d'oléfine, les esters de phosphate, l'acide stéarique et les mélanges et copolymères de ceux-ci. La préférence est donnée à l'utilisation d'au moins deux liants, et le liant préféré est composé de cire de paraffine, de cire de polyéthylène et d'acide stéarique.

8. Procédé selon l'une quelconque des précédentes revendications, dans lequel l'élimination du liant par voie thermique est effectuée à une température inférieure à 500°C.

9. Procédé selon l'une quelconque des précédentes revendications, dans lequel le frittage est effectué à 50 à 99% du point de fusion du métal ou de l'alliage métallique.

10. Procédé selon l'une quelconque des revendications 3 à 9, dans lequel le mélange de poudre de titane ou de poudre d'alliage de titane et d'un liant polymère ; et/ou de poudre de magnésium ou de poudre d'alliage de magnésium et d'un liant polymère est de préférence effectué, indépendamment, dans un malaxeur, une extrudeuse, un malaxeur à rotation ou un malaxeur à résonance.

11. Procédé selon la revendication 10, dans lequel le mélange est effectué à une température de 50 à 250°C.

12. Procédé selon l'une quelconque des précédentes revendications, dans lequel le moulage par injection est effectué, indépendamment, à une température de fusion de 50 à 250°C.

13. Procédé selon l'une quelconque des précédentes revendications, dans lequel le moulage par injection est effectué, indépendamment, à une pression de 40 à 200 MPa.
